# FASCICULE DE BREVET EUROPEEN

(11) **EP 1 720 599 B1**
(45) Date de publication et mention de la délivrance du brevet: **16.09.2015**
(21) Numéro de dépôt: 05733010.2
(22) Date de dépôt: 02.03.2005
(51) Int. Cl.: A61M 39/10, A61M 25/10, A61M 25/06, A61M 39/06

(54) **DISPOSITIF A ORGANE DE SECURITE COULISSANT POUR LA MISE EN PLACE D'UNE CANULE DANS UNE VEINE**
VORRICHTUNG MIT EINEM VERSCHIEBBAREN SICHERHEITSELEMENT ZUR POSITIONIERUNG EINER KANÜLE IN EINER VENE
DEVICE WITH A SLIDABLE SAFETY MEMBER FOR POSITIONING A CANNULA IN A VEIN

(30) Priorité: 02.03.2004 FR 0402121
(43) Date de publication de la demande: 15.11.2006
(73) Titulaire: Vygon, 95440 Ecouen (FR)
(72) Inventeur: CARREZ, Jean-Luc, F-95440 Ecouen (FR); GUYOMARC'H, Pierrick, F-95120 Ermont (FR); DALLE, Valéry, F-60270 Gouvieux (FR); HUET, Jean-Max, F-92110 Clichy (FR)
(74) Mandataire: Regimbeau
(86) Numéro de dépôt international: PCT/FR2005/000493
(87) Numéro de publication internationale: WO 2005/087306

(56) Documents cités:
- EP-A- 0 832 666
- EP-A- 1 378 263
- EP-A2- 0 747 083
- WO-A1-96/22800
- US-A- 4 900 307
- US-A- 5 573 510
- US-A- 5 575 777
- US-A- 5 695 474
- US-B1- 6 749 588

## Description

La présente invention concerne les dispositifs utilisés pour la mise en place dans une veine d'une canule constituée d'un cathéter tubulaire court à embase proximale, au moyen d'une aiguille de ponction.

La procédure d'introduction comprend une phase de ponction dans laquelle l'aiguille est poussée dans l'embase du cathéter et dans le cathéter en sorte que sa pointe sorte à l'extrémité distale du cathéter et dans laquelle l'opérateur ponctionne avec cette pointe la veine dans laquelle il veut introduire le cathéter, une phase d'introduction dans laquelle l'opérateur fait glisser le cathéter sur l'aiguille en direction distale pour faire pénétrer le cathéter dans la veine, et une phase de retrait dans laquelle l'opérateur retire l'aiguille de la veine, du cathéter et de l'embase du cathéter.

A l'issue de la phase de retrait, la pointe de l'aiguille se trouve à l'air libre et le risque se présente que l'opérateur qui tient le cathéter et son embase d'une main et qui tient l'aiguille de l'autre main, contrôle mal l'aiguille et se pique avec sa pointe.

La publication EP 0832 666 décrit un dispositif qui comporte une pièce intermédiaire entre l'embase d'une canule et l'extrémité d'un tube qui sert à piéger l'aiguille.

La pièce intermédiaire est conçue à une extrémité pour le montage de la pièce sur l'embase de la canule et à son extrémité opposée pour le montage sur la pièce du tube destiné à piéger l'aiguille.

La pièce intermédiaire n'est pas destinée à piéger l'aiguille mais à fournir un joint d'étanchéité.

Le montage de l'embase de la canule sur cette pièce est réalisé par vissage ou par coincement conique, ce qui n'interdit pas que la pièce soit détachée de cette embase par maladresse avant que l'aiguille soit retirée.

Si ce dispositif présente un moyen d'accrochage provisoire du tube qui entoure l'aiguille sur la pièce intermédiaire, cet accrochage ne concerne pas la fixation provisoire de la pièce intermédiaire sur l'embase de la canule.

La publication US 4 900 307 décrit un dispositif qui est seulement prévu pour retirer l'aiguille à l'intérieur d'un tube à l'extrémité duquel est fixé un nez. Ce nez après le montage de l'aiguille dans le dispositif est fixé de façon permanente sur l'extrémité du tube. Le dispositif comporte une gâchette actionnable manuellement pour déclencher le retrait de l'aiguille.

Il est également connu de fixer provisoirement dans le prolongement vers l'arrière de l'embase du cathéter une cage détachable au travers de laquelle l'aiguille peut coulisser et qui est munie d'un piège pour retenir dans la chambre l'extrémité de ponction de l'aiguille lorsque celle-ci sort de l'embase et pour rester en place sur cette extrémité lorsque la cage est détachée de l'embase voir document EP 0 747 083.

Pour fixer provisoirement la cage sur l'embase de l'aiguille, il est connu de réaliser un emboîtement conique à friction de la cage dans ou sur l'embase de l'aiguille, en sorte que la cage se détache de l'embase sous l'effet d'une traction exercée axialement sur l'aiguille après que l'extrémité de ponction de l'aiguille soit arrivée dans la cage (EP 0 456 694 ou US 5 322 517, US 5 135 504, US 5 176 655, et autres).

Le risque subsiste toutefois que la cage se détache de l'embase prématurément avant que l'extrémité de ponction de l'aiguille soit piégée dans la cage.

Pour éviter ce risque, il a été préconisé de munir la cage d'un organe mobile transversalement, maintenu par l'aiguille dans une position où il est retenu par l'embase du cathéter, et apte à venir de lui-même dans une position de libération lorsque l'extrémité de ponction de l'aiguille est retirée dans la cage.

La publication EP 0 891 198 ou US 6 001 080 réalise cette retenue par pénétration, dans une cavité formée sur la face interne de la paroi de l'embase du cathéter, d'un bec formé sur une paroi de la cage, ledit bec étant maintenu en position de retenue par un contact latéral avec l'aiguille et se trouvant libéré et apte à se déplacer radialement pour échapper à la cavité lorsque ce contact latéral est supprimé par le retrait de l'aiguille en arrière du bec.

Ce dispositif de retenue, entièrement caché dans l'embase et dans la cage, est difficile à contrôler et le déplacement radial automatique de l'ergot peut être insuffisant pour libérer la cage de l'embase.

La publication US 6 234 999 décrit un autre dispositif de retenue dans lequel la cage présente un externe pourvu d'un bec retenu par une collerette externe de l'embase mais qui n'est pas maintenu par l'aiguille, en sorte qu'une traction intempestive sur la cage risque de supprimer prématurément la retenue.

La présente invention a pour but de fournir une cage muni d'un dispositif de retenue affranchi des inconvénients précités et notamment un dispositif de retenu qui soit à la fois apparent à l'extérieur de la cage et de l'embase et maintenu par l'aiguille en position de retenue tant que l'extrémité de ponction de l'aiguille n'est pas retirée dans la cage.

Un objet de l'invention est donc un dispositif pour la mise en place dans une veine d'une canule constitué d'un cathéter tubulaire court à embase proximale, ce dispositif comprenant une aiguille qui présente une extrémité de ponction et une cage qui prolonge l'embase de la canule en direction proximale, cette cage déterminant une chambre traversée à coulisse par l'aiguille d'une entrée proximale à une sortie distale et munie d'un piège pour retenir dans la chambre l'extrémité de ponction de l'aiguille lorsque l'aiguille est extraite de l'embase du cathéter, la cage et l'embase étant munis de moyens de retenue coopérant pour assurer une retenue provisoire de la cage sur l'embase avant que l'extrémité de ponction de l'aiguille soit piégée dans la chambre de la cage, lesdits moyens de retenue comprenant un rebord externe formé sur l'embase et un bec externe prévu sur la cage pour être retenue par ce rebord, caractérisé en ce que le bec est formé sur un organe mobile qui comporte une paroi traversée par un trou pour le passage de l'aiguille en sorte que le passage de l'aiguille dans le trou maintienne le bec en position de retenue, ladite paroi étant montée à coulisse dans la chambre de la cage selon une direction transversale à l'aiguille entre une position basse de retenue et une position haute de libération en sorte que lorsque l'aiguille est retirée dans la cage jusqu'à être en retrait dudit trou, la paroi qui n'est pas traversée par l'aiguille peut coulisser vers sa position haute, ce qui soulève ledit bec et permet de séparer la cage de l'embase du cathéter.

Dans des réalisations particulières, le dispositif de l'invention présente encore une ou plusieurs des caractéristiques suivantes :
- le bec est formé à l'extrémité d'une paroi de l'organe de sécurité qui est d'équerre avec la paroi coulissante ;
- la collerette et/ou le bec présente une rampe en sorte qu'un recul de la cage en direction proximale provoque le soulèvement du bec lorsque l'aiguille ne traverse plus ledit trou;
- des moyens sont prévus pour limiter le soulèvement de la paroi coulissante en position haute ;
- des moyens sont prévus pour empêcher que l'organe mobile revienne en position basse après être venu en position haute ;
- la cage est formée d'une pièce arrière qui constitue la chambre et l'entrée de la chambre et d'une pièce avant qui comporte une paroi transversale qui ferme ladite chambre et qui comporte la sortie de la chambre, cette paroi transversale déterminant une coulisse pour ladite paroi coulissante dudit organe mobile, ces deux pièces étant maintenues assemblées par encliquetage de tétons prévus sur les côtés de la paroi transversale de la pièce avant dans des trous formés dans la paroi de la chambre de la pièce arrière, ou inversement ;
- la pièce avant de la cage constitue en avant de ladite paroi transversale un nez apte à être reçu dans l'embase du cathéter ;
- la paroi transversale de la pièce avant de la cage porte un téton qui coulisse dans une lumière oblongue de la paroi coulissante de l'organe mobile pour limiter le soulèvement de l'organe mobile ;
- la paroi coulissante de l'organe mobile présente des pattes latérales souples et élastiques qui sont contraintes par des rebords formés sur la paroi transversale de la pièce avant de la cage tant que l'organe n'est pas arrivé en position haute et qui se déploient, lorsque de l'organe mobile arrive en position haute, pour reposer sur ces rebords en empêchant que de l'organe mobile revienne en position basse ;
- la pièce arrière de la cage est conformée pour que l'embase de l'aiguille s'emboîte sur cette pièce pour la ponction ;
- des moyens sont prévus pour empêcher que la pointe de l'aiguille puisse ressortir par l'entrée de la chambre.

On décrira ci-après un exemple de réalisation non limitatif de la portée de l'invention en référence aux figures du dessin joint sur lequel :

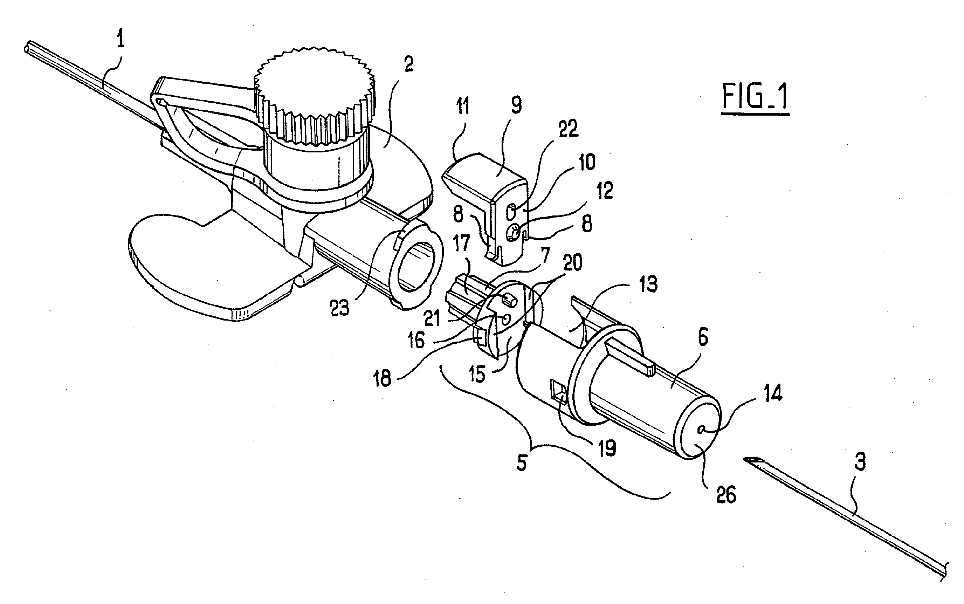

- la figure 1 est une vue en perspective du dispositif dans laquelle le cathéter court avec son embase, l'aiguille, les deux parties de la cage et l'organe de retenue coulissant ont été représentés séparés ;
- les figures 2 et 3 représentent le dispositif prêt pour l'emploi, respectivement en perspective avec arrachement et en coupe axiale ;

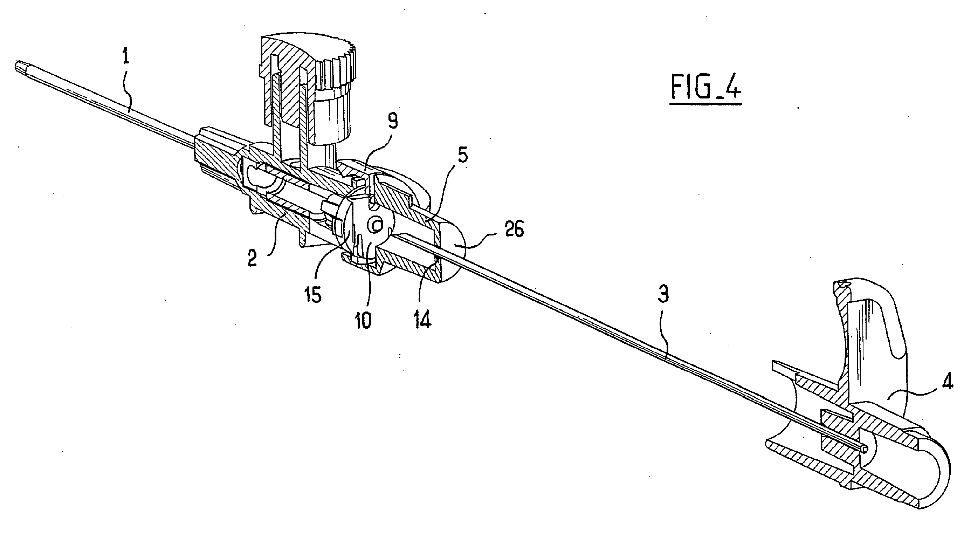

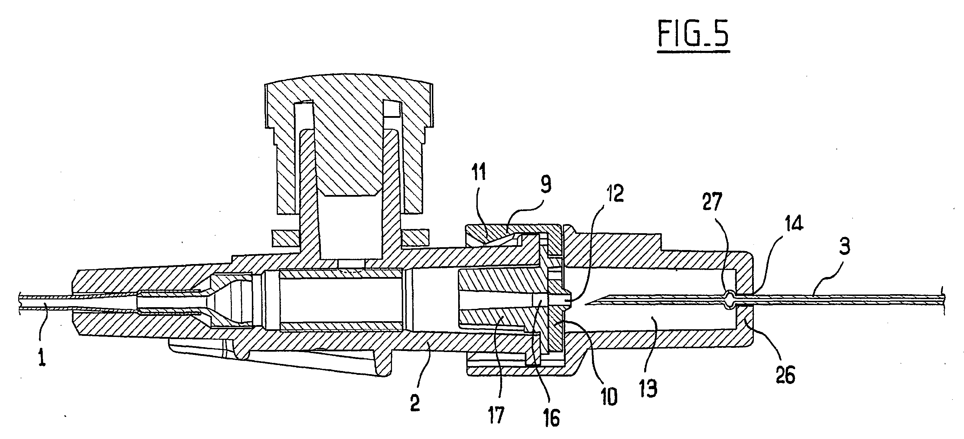

- les figures 4 et 5 représentent le dispositif, respectivement en perspective avec arrachements et en coupe axiale, alors que la pointe de l'aiguille est retirée dans la chambre de la cage ;
- les figures 6 et 7 représentent le dispositif, respectivement en perspective avec arrachements et en coupe axiale, alors que l'organe de retenue est en cours de soulèvement ;
- la figure 8 est une vue agrandie d'un détail de la figure 6 ;

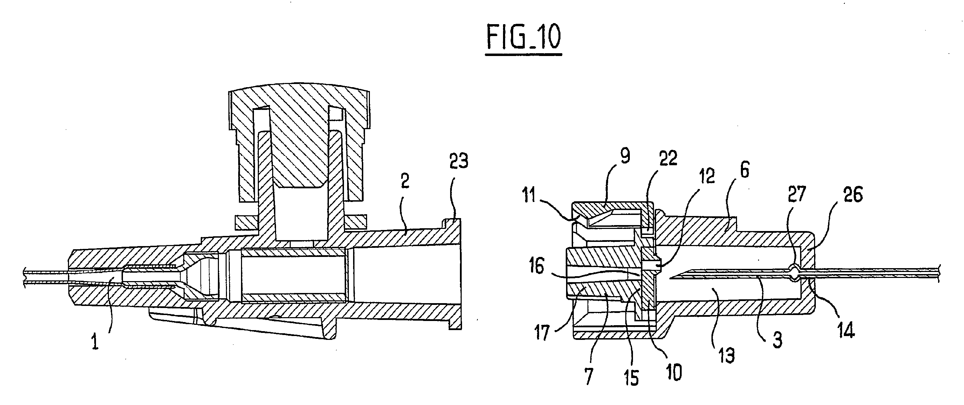

- les figures 9 et 10 représentent le dispositif, respectivement en perspective avec arrachements et en coupe axiale, alors que la cage est détachée de l'embase du cathéter ;
- la figure 11 est une vue agrandie d'un détail de la figure 9, et
- la figure 12 est une vue en plan de la paroi transversale de la pièce avant masquée en partie par la paroi coulissante de l'organe de retenue.

Le dispositif représenté sur les figures comprend :
- un cathéter court tubulaire (1) muni d'une embase proximale (2) ;
- une aiguille (3) qui présente une extrémité de ponction (3a) et qui est munie d'une embase proximale (4) ;
- une cage (5) constituée d'une pièce arrière (6) et d'une pièce avant (7) ;
- un organe de retenue mobile (8).

L'organe de retenue est un corps moulé qui présente deux parois (9 ; 10) disposées à l'équerre. L'une des parois (9) se termine par un bec (11) et l'autre paroi (10), qui est percée d'un trou (12) pour le passage de l'aiguille.

La pièce arrière (6) de la cage détermine une chambre (13) pour le passage de l'aiguille à travers la cage et détermine l'entrée (14) de cette chambre tandis que la pièce avant (7) de la cage détermine une paroi transversale (15) pour fermer la chambre, cette paroi étant percée d'un trou (16) qui constitue la sortie de la chambre. A l'avant de cette paroi, la pièce avant (7) détermine un nez (17) apte à être reçu dans l'embase du cathéter.

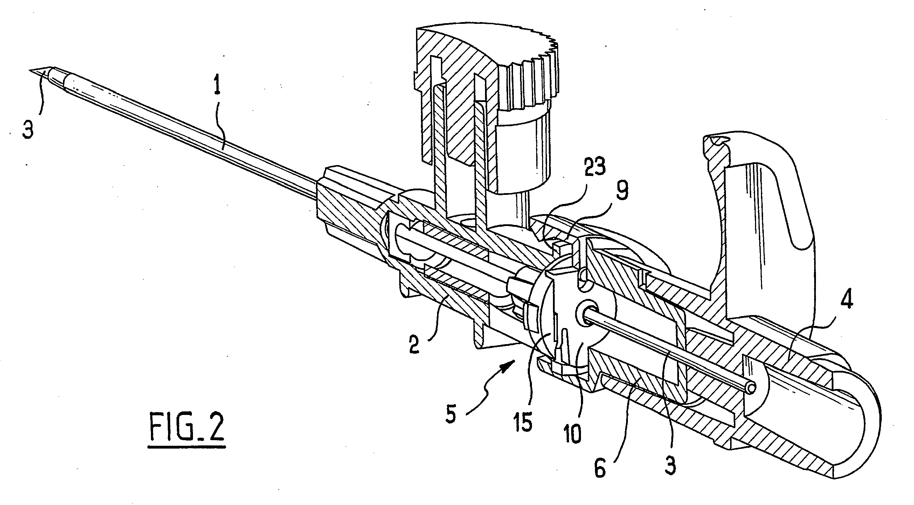

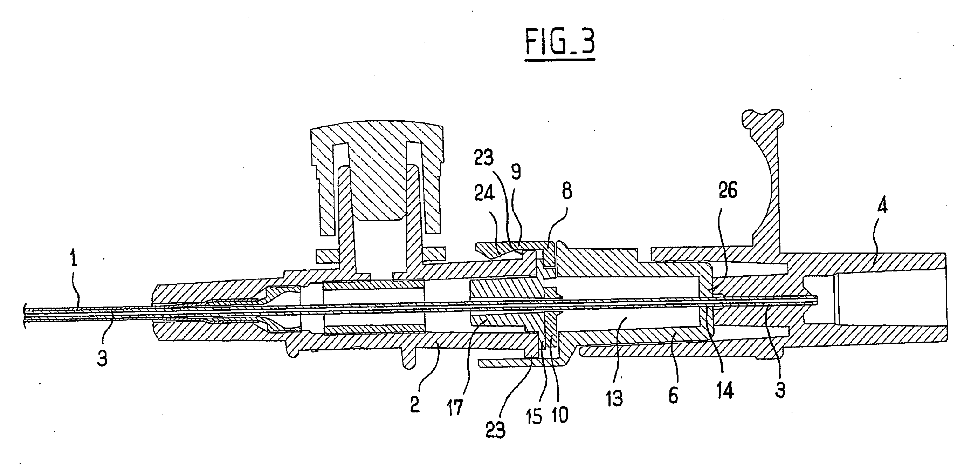

La pièce arrière (6) de la cage est conformée pour que l'embase de l'aiguille s'emboîte sur la pièce arrière pour la ponction (figures 2 et 3).

La paroi transversale (15) de la pièce avant présente des tétons latéraux (18) aptes à s'encliqueter dans des trous (19) de la paroi de la chambre de la pièce arrière pour la fixation des deux pièces.

Cette paroi transversale détermine également des glissières (20) pour le coulissement de la paroi (10) de l'organe de retenue.

Enfin, cette paroi transversale est munie d'un téton (21) apte à être reçu dans une lumière oblongue (22) de la paroi coulissante (10) pour limiter le soulèvement de l'organe mobile.

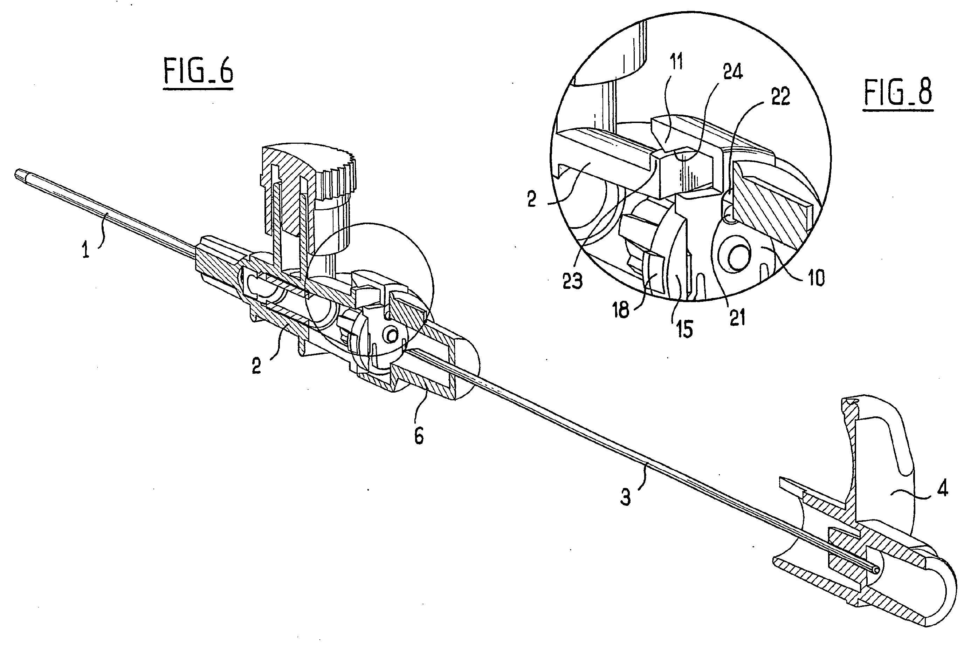

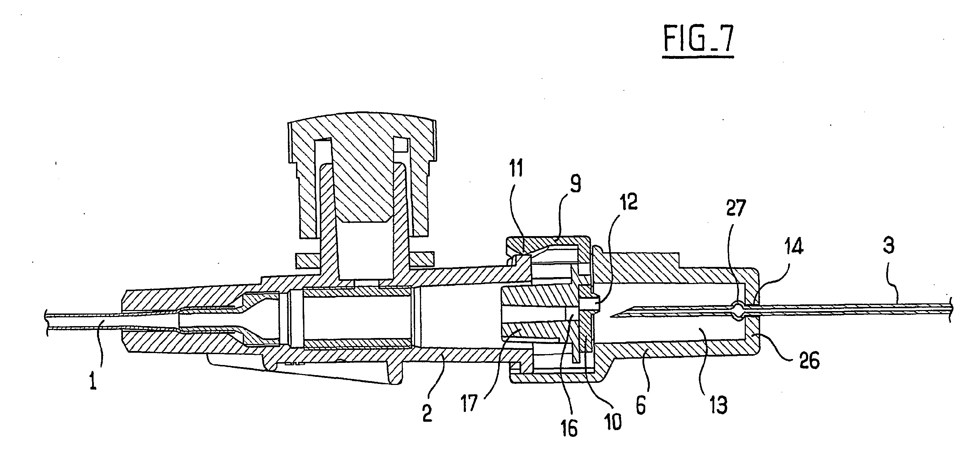

L'embase (2) du cathéter court présente à proximité de son entrée un rebord (23) par exemple une collerette externe continue ou discontinue, pour arrêter le bec (11) de la guillotine et ce bec présente une rampe (24) apte à glisser progressivement sur la collerette lorsque la cage est tirée en direction proximale (figures 6 à 8), ce qui provoque le soulèvement de la guillotine jusqu'à ce que ce soulèvement soit arrêté par le téton (21) comme on le voit notamment sur la figure 8. Les positions du téton (21) et de la lumière (22) sont réglées pour que cet arrêt ne soit pas obtenu avant que le bec soit passé au dessus de la collerette (figure 7).

Des moyens sont prévus pour empêcher que la guillotine revienne en position basse après être arrivée en position levée.

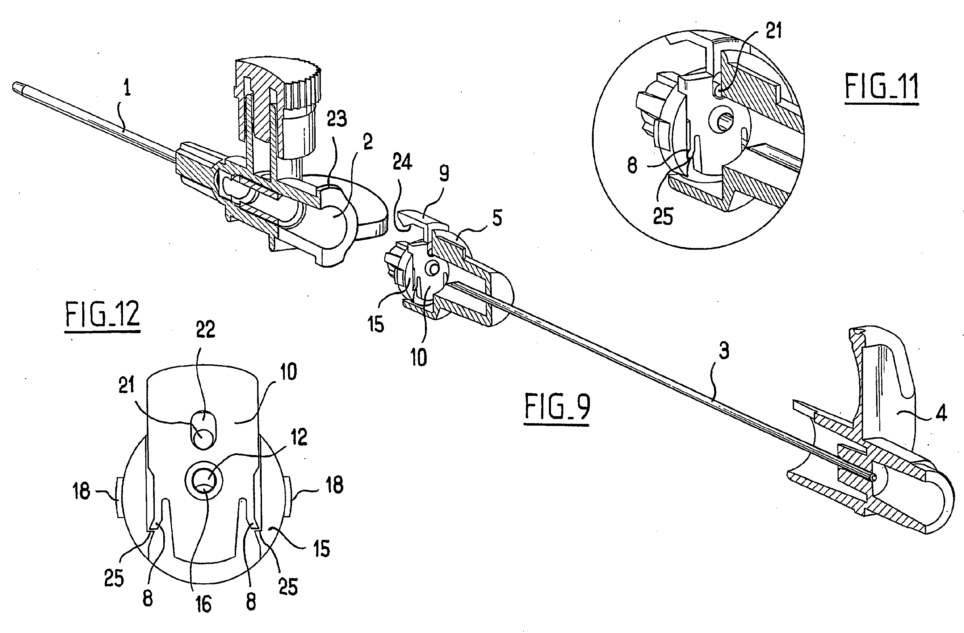

Dans l'exemple représenté, ces moyens sont constitués par des pattes souples et élastiques (8) formées sur les côtés de la paroi coulissante (10) de l'organe mobile et qui sont contraintes par des rebords (25) de la paroi transversale (15) de la pièce avant (7) de la cage jusqu'à ce que ces pattes soient arrivées au dessus des rebords lors du soulèvement de la guillotine (figures 11 et 12) position pour laquelle les pattes se détendent vers l'extérieur et reposent sur les rebords.

De préférence, des moyens sont également prévus, de façon en soi connue pour empêcher que l'extrémité de ponction de l'aiguille puisse sortir de la cage par l'entrée proximale de la chambre.

On a proposé de relier la cage à l'embase de l'aiguille par une liaison déployable telle que à l'état déployée cette liaison ait une longueur inférieur à la longueur de l'aiguille (WO 94/00172, US 5 176 655, US 6 234 999, US 6 001 080).

On a également proposé de limiter l'entrée proximale de la cage à un simple trou pour le passage de l'aiguille et de munir l'aiguille d'un renflement local en sorte que le coulissement de l'aiguille en direction proximale soit arrêté par butée de ce renflement contre le pourtour du trou.

La présente invention ne porte pas en soi sur un choix particulier d'un tel dispositif et, pour l'exemple uniquement, on a représenté un dispositif constitué par une paroi transversale fixe (26) dans laquelle est percée l'entrée proximale (14) de la cage et par un renflement local (27) de la section droite de l'aiguille. Ce renflement n'a été représenté que sur certaines figures et de façon exagérée : il va de soi que ce renflement est conçu pour ne pas empêcher le glissement de l'aiguille dans la canule.

L'invention n'est pas limitée aux réalisations qui ont été décrites.

## Revendications

1. Dispositif pour la mise en place dans une veine d'une canule constitué d'un cathéter (1) court à embase proximale (2), ce dispositif comprenant une aiguille (3) qui présente une extrémité de ponction (3a) et une cage (5) qui prolonge l'embase de la canule en direction proximale, cette cage déterminant une chambre (13) traversée à coulisse par l'aiguille d'une entrée proximale (14) à une sortie distale (16) et munie d'un piège pour retenir dans la chambre l'extrémité de ponction de l'aiguille lorsque l'aiguille est extraite de l'embase du cathéter, la cage et l'embase étant munis de moyens de retenue coopérant pour assurer une retenue provisoire de la cage sur l'embase avant que l'extrémité de ponction de l'aiguille soit piégée dans la chambre de la cage, lesdits moyens de retenue comprenant un rebord externe (23) formé sur l'embase et un bec (11) externe prévu sur la cage pour être retenue par ce rebord, le bec étant formé sur un organe mobile (9, 10) qui comporte une paroi (10) traversée par un trou (12) pour le passage de l'aiguille en sorte que le passage de l'aiguille dans le trou maintienne le bec en position de retenue **caractérisé en ce que** ladite paroi (10) est montée à coulisse dans la chambre de la cage selon une direction transversale à l'aiguille entre une position basse de retenue et une position haute de libération en sorte que lorsque l'aiguille est retirée dans la cage jusqu'à être en retrait dudit trou, la paroi (10) qui n'est pas traversée par l'aiguille peut coulisser vers sa position haute, ce qui soulève ledit bec et permet de séparer la cage de l'embase du cathéter, ledit rebord (23) et/ou ledit bec (11) présentant une rampe (24) en sorte qu'un recul de la cage en direction proximale provoque le soulèvement du bec lorsque l'aiguille (3) ne traverse plus ledit trou (12).

2. Dispositif selon la revendication 1, dans lequel ledit bec (11) est formé à l'extrémité d'une paroi (9) de l'organe de retenue qui est d'équerre avec la paroi coulissante (10).

3. Dispositif selon l'une des revendications 1 à 2, et qui comporte des moyens (21; 22) pour limiter le soulèvement de la paroi coulissante en position haute.

4. Dispositif selon l'une des revendications 1 à 3, et qui comporte des moyens (8; 8) pour empêcher que l'organe mobile revienne en position basse après être venu en position haute.

5. Dispositif selon l'une des revendications 1 à 4, dont la cage (5) est formée d'une pièce arrière (6) qui constitue la chambre (13) et l'entrée (14) de la chambre et d'une pièce avant (7) qui comporte une paroi transversale (15) qui ferme ladite chambre et qui comporte la sortie (16) de la chambre, cette paroi transversale (15) déterminant une coulisse pour ladite paroi coulissante (10) dudit organe de retenue, ces deux pièces (6,7) étant maintenues assemblées par encliquetage de tétons (18) prévus sur les côtés de la paroi transversale de la pièce avant dans des trous (19) formés dans la paroi de la chambre de la pièce arrière, ou inversement.

6. Dispositif selon la revendication 5 dans lequel la pièce avant (7) de la cage constitue en avant de ladite paroi transversale (15) un nez (17) apte à être reçu dans l'embase du cathéter.

7. Dispositif selon l'une des revendications 4 à 6, dans lequel ladite paroi transversale (15) de la pièce avant (7) de la cage porte un téton (21) qui coulisse dans une lumière oblongue (22) de la paroi coulissante de l'organe de retenue pour limiter le soulèvement de l'organe mobile.

8. Dispositif selon l'une des revendications 4 à 7 et dans lequel ladite paroi coulissante (10) de l'organe de retenue présente des pattes latérales souples et élastiques (8, 8) qui sont contraintes par des rebords (25) formés sur la paroi transversale (15) de la pièce avant (7) de la cage tant que ledit organe n'est pas arrivé en position haute et qui se déploient, lorsque de l'organe arrive en position haute, pour reposer sur ces rebords en empêchant que de l'organe revienne en position basse.

9. Dispositif selon l'une des revendications 4 à 8 dans lequel la pièce arrière de la cage est conformée pour que l'embase de l'aiguille s'emboîte sur cette pièce pour la ponction.

10. Dispositif selon l'une des revendications 1 à 9 et qui comporte des moyens (14, 27) pour empêcher que la pointe de l'aiguille puisse ressortir par l'entrée de la chambre.

## Patentansprüche

1. Vorrichtung für das Einsetzen einer aus einem kurzen Katheter (1) mit proximaler Basis (2) bestehenden Kanüle in eine Vene, wobei diese Vorrichtung eine Nadel (3) umfasst, die ein Punktierende (3a) und einen Käfig (5) aufweist, die die Basis der Kanüle in proximaler Richtung verlängert, wobei dieser Käfig eine Kammer (13) bestimmt, die gleitend durch die Nadel von einem proximalen Eingang (14) zu einem distalen Ausgang (16) durchquert wird und mit einer Falle versehen ist, um das Punktierende der Nadel in der Kammer zurückzuhalten, wenn die Nadel aus der Basis des Katheters herausgezogen ist, wobei der Käfig und die Basis mit Rückhaltemitteln versehen sind, die zusammenwirken, um ein vorübergehendes Zurückhalten des Käfigs auf der Basis zu gewährleisten, bevor das Punktierende der Nadel in der Kammer des Käfigs festgehalten wird, wobei die genannten Rückhaltemittel einen auf der Basis geformten externen Rand (23) und einen auf dem Käfig vorgesehenen externen Schnabel (11) umfassen, um von diesem Rand zurückgehalten zu werden, wobei der Schnabel auf einem mobilen Organ (9, 10) geformt ist, das eine Wand (10) umfasst, die durch ein Loch (12) für den Durchgang der Nadel derart durchquert wird, dass der Durchgang der Nadel in dem Loch den Schnabel in der Festhalteposition hält, **dadurch gekennzeichnet, dass** die genannte Wand (10) gemäß einer Querrichtung zur Nadel zwischen einer niedrigen Rückhalteposition und einer hohen Freisetzungsposition gleitend in der Kammer des Käfigs derart montiert ist, dass, wenn die Nadel in dem Käfig zurückgezogen ist, bis sie von dem genannten Loch zurückgezogen ist, die Wand (10), die nicht von der Nadel durchquert wird, zu ihrer oberen Position gleiten kann, was den genannten Schnabel anhebt und die Trennung des Käfigs von der Basis des Katheters zulässt, wobei der genannte Rand (23) und / oder der genannte Schnabel (11) eine Rampe (24) derart aufweist / aufweisen, dass ein Rückzug des Käfigs in proximaler Richtung das Anheben des Schnabels hervorruft, wenn die Nadel (3) das genannte Loch (12) nicht mehr durchquert.

2. Vorrichtung gemäß Anspruch 1, in dem der genannte Schnabel (11) am Ende einer Wand (9) des Rückhalteorgans geformt ist, das mit der gleitenden Wand (10) einen Winkel bildet.

3. Vorrichtung gemäß Anspruch 1 bis 2 und die Mittel (21; 22) umfasst, um das Anheben der gleitenden Wand in hoher Position zu begrenzen.

4. Vorrichtung gemäß Anspruch 1 bis 3 und die Mittel (8; 8) umfasst, um zu verhindern, dass das mobile Organ in die niedrige Position zurückkehrt, nachdem sie in die hohe Position gekommen ist.

5. Vorrichtung gemäß Anspruch 1 bis 4, deren Käfig (5) aus einem hinteren Teil (6), der die Kammer (13) und den Eingang (14) der Kammer formt, und aus einem vorderen Teil (7), der eine Querwand (15) umfasst, die die genannte Kammer schließt und die den Ausgang (16) der Kammer umfasst, gebildet ist, wobei diese Querwand (15) ein Gleiten für die genannte gleitende Wand (10) des genannten Rückhalteorgans bestimmt, wobei diese zwei Teile (6, 7) per Einrasten von Ansätzen (18) zusammengebaut gehalten werden, die auf den Seiten der Querwand des vorderen Teils in den Löchern (19) vorgesehen sind, die in der Wand der Kammer des hinteren Teils oder umgekehrt geformt sind.

6. Vorrichtung gemäß Anspruch 5, bei dem das vordere Teil (7) des Käfigs vor der genannten Querwand (15) eine Nase (17) bildet, die geeignet ist, in der Basis des Katheters aufgenommen zu werden.

7. Vorrichtung gemäß Anspruch 4 bis 6, in der die genannte Querwand (15) des vorderen Teils (7) des Käfigs einen Ansatz (21) trägt, der in einer länglichen Öffnung (22) der gleitenden Wand des Rückhalteorgans gleitet, um das Anheben des mobilen Organs zu begrenzen.

8. Vorrichtung gemäß Anspruch 4 bis 7 und in der die genannte gleitende Wand (10) des Rückhalteorgans elastische und weiche laterale Klauen (8, 8) aufweist, die durch die auf der Querwand (15) des vorderen Teils (7) des Käfigs geformten Ränder (25) eingespannt sind, solange das genannte Organ nicht in der hohen Position eingetroffen ist, und die, wenn das Organ in der hohen Position eintrifft, sich spreizen, um auf diesen Rändern zu ruhen und dabei zu verhindern, dass das Organ in die niedrige Position zurückkehrt.

9. Vorrichtung gemäß Anspruch 4 bis 8, in der das hintere Teil des Käfigs angepasst ist, damit die Basis der Nadel auf diesem Teil zum Punktieren einrastet.

10. Vorrichtung gemäß Anspruch 1 bis 9 und die Mittel (14, 27) umfasst, um zu verhindern, dass die Spitze der Nadel durch den Eingang der Kammer wieder heraustreten kann.

## Claims

1. A device for placing in a vein a cannula made of a short catheter (1) with a proximal hub (2), this device comprising a needle (3) having a puncture end (3a) and a cage (5) which extends the hub of the cannula in a proximal direction, this cage determining a chamber (13) slidingly crossed by the needle from a proximal entry (14) to a distal exit (16) and fitted with a trap to hold back in the chamber the needle puncture end when the needle is extracted from the catheter hub, the cage and the hub being provided with holding means cooperating to ensure a temporary holding of the cage on the hub before the needle puncture end is trapped in the cage chamber, said holding means comprising an outer rim (23) formed on the hub and an outer mouth (11) provided on the cage to be held by this rim, the mouth being formed on a moveable member (9, 10) which has a wall (10) crossed by a hole (12) for passing the needle so that the needle passing in the hole keeps the mouth in a holding position, **characterized in that** said wall (10) is slidingly mounted in the cage chamber along a direction transverse to the needle between a holding low position and a release upper position such that when the needle is removed from the cage up to being recessed from said hole, the wall (10) which is not crossed by the needle can slide towards its upper position, which raises said mouth and enables the cage to be separated from the catheter hub, said rim (23) and/or said mouth (11) having a ramp (24) such that a backward movement of the cage in a proximal direction causes the raising of the mouth when the needle (3) does no longer cross said hole (12).

2. The device according to claim 1, wherein said mouth (11) is formed at the end of a wall (9) of the holding member which is normal to the sliding wall (10).

3. The device according to one of claims 1 to 2, and which comprises means (21; 22) for limiting the raising of the sliding wall in an upper position.

4. The device according to one of claims 1 to 3, and which comprises means (8; 8) for preventing the moveable member from coming back in a low position after it has come in an upper position.

5. The device according to one of claims 1 to 4, the cage (5) of which is formed by a rear piece (6) which makes up the chamber (13) and the entry (14) of the chamber and of a front piece (7) which comprises a transverse wall (15) which closes said chamber and which comprises the exit (16) of the chamber, this transverse wall (15) determining a slider for said sliding wall (10) of said holding member, both these pieces (6, 7) being kept assembled by snap fitting dog points (18) provided on the sides of the transverse wall of the front piece in holes (19) formed in the chamber wall of the rear piece, or conversely.

6. The device according to claim 5, wherein the front piece (7) of the cage makes up at the front of said transverse wall (15) a nose (17) apt to be received in the catheter hub.

7. The device according to one of claims 4 to 6, wherein said transverse wall (15) of the front piece (7) of the cage carries a dog point (21) which slides in an oblong port (22) of the sliding wall of the holding member to limit the raising of the moveable member.

8. The device according to one of claims 4 to 7, and wherein said sliding wall (10) of the holding member has flexible elastic side legs (8, 8) which are constrained by rims (25) formed on the transverse wall (15) of the front piece (7) of the cage as long as said member has not reached the upper position and which expand, when the member reaches the upper position, to rest on these rims by preventing the member from coming back in the low position.

9. The device according to one of claims 4 to 8, wherein the rear piece of the cage is shaped so that the needle hub engages the latter for the puncture.

10. The device according to one of claims 1 to 9, and which comprises means (14, 27) for preventing the tip of the needle from being able to exit via the chamber entry.
